# EUROPEAN PATENT SPECIFICATION

(11) **EP 2 197 506 B1**
(45) Date of publication and mention of the grant of the patent: **13.06.2012**
(21) Application number: 07819164.0
(22) Date of filing: 19.10.2007
(51) Int. Cl.: A61L 27/56, A61L 27/58, A61L 27/46

(54) **SYNTHETIC BONE SUBSTITUTE, METHOD FOR PREPARING SAME AND METHOD FOR FILLING A CAVITY IN A SUBSTRATE**
SYNTHETISCHES KNOCHENSUBSTITUT, VERFAHREN ZU SEINER HERSTELLUNG UND VERFAHREN ZUM AUFFÜLLEN EINES HOHLRAUMS IN EINEM SUBSTRAT
SUBSTITUT OSSEUX SYNTHÉTIQUE, SON PROCÉDÉ DE PRÉPARATION ET PROCÉDÉ POUR COMBLER UNE CAVITÉ DANS UN SUBSTRAT

(43) Date of publication of application: 23.06.2010
(73) Proprietor: Stryker Trauma GmbH, 24232 Schönkirchen (DE)
(72) Inventor: PROCTER, Philip, F-01220 Divonne les Bains (FR)
(74) Representative: Kühn, Ralph
(86) International application number: PCT/EP2007/009099
(87) International publication number: WO 2009/049650

(56) References cited:
- WO-A1-00/20353
- WO-A2-2007/068489
- GB-A- 2 365 423
- US-A1- 2003 039 676
- US-A1- 2003 135 284
- US-A1- 2006 088 601
- MATHIEU L.M., BOURBAN P.E., MANSON J.A.E: "Processing of homogeneous ceramic/polymer blends for bioresorbable composites" COMPOSITES SCIENCE AND TECHNOLOGY, vol. 66, no. 11-12, 2006, pages 1606-1614, XP002546911

## Description

### Field of the invention

The present invention relates to a synthetic bone substitute which may be inserted into bony voids or cavities, to a method for preparing such synthetic bone substitute and to a method for filling a cavity in a substrate.

### Technical background

Filling of bony voids and cavities for example after a bony fracture, breakage or damage may be an important challenge in the medical device area. For example due to an accident cavities may be formed in a human bone. To accelerate a healing process or to stabilize the cavity, the cavity may be filled with a bone substitute.

One possible prior art approach is to harvest bone from the patient at a different location of the body and then insert the harvested bone material into the cavity. A problem therein may be that the cavity to be filled is originally irregular. Before insertion of the harvested bone, both the cavity and the harvested bone may have to be shaped into a corresponding form such as to fit the harvested bone into the cavity. A further problem may be that the cavity may have a larger cross-section than an original entry hole to the cavity. In order to be able to insert the bone substitute the entry hole has to be enlarged to a dimension similar to the cross section of the cavity. Such enlargement of the entry hole may further weaken the already damaged bone.

As an alternative, synthetic bone graft materials have been developed that are injectable and enable irregular cavities to be filled. These are commonly calcium phosphate based cements. Due to their viscosity or fluidity, these cements can completely fill the cavity and the form of the cement is easily formed by the cavity itself. However, these cements do not possess the strength of cortical bone or a porosity that is equivalent to cancellous bone.

WO 2007/068489 A2 discloses a moldable biomaterial for bone regeneration. WO 00/20353 discloses a method for producing a macro-porous ceramic foam. US 2003/0039676 A1 discloses a shaped load-bearing osteo-implant and methods of making same. US 2006/088601 A1 discloses a synthetic bone substitute material.

### Summary of the invention

There may be a need to provide an improved bone substitute which overcomes at least some of the above described drawbacks of prior art approaches at least in part. Particularly, there may be a need to provide a bone substitute which, on the one hand, can be easily inserted into an irregular bone cavity and which, on the other hand, may exhibit sufficient strength and/or porosity. Furthermore, there may be a need for a method for preparing such bone substitute and a method for filling a cavity in a substrate using such synthetic bone substitute.

These needs may be met by the subject-matter of the independent claims. Embodiments of the invention are described in the dependent claims.

According to a first aspect of the invention a synthetic bone substitute comprises a porous foam structure comprising a bio-resorbable polymer and a bio-ceramic filler material. The bone substitute further comprises a plasticizer for softening the porous foam structure.

It may be seen as a gist of the present invention that the basis for the synthetic bone substitute is a scaffold made of a bio-resorbable polymer that has been blended with a bio-ceramic. The blend is manufactured to have a porous foam structure. The bone substitute is softened by using a plasticizer. The softened bone substitute mass can then be compressed and introduced into a bony cavity for example through a cannula. Accordingly, a number of pieces of the bone substitute can be introduced into a bone void. The bone substitute pieces may then expand to its original volume and in the presence of blood and/or other body fluids, the plasticizer may be absorbed into these fluids and the bone substitute may return to its original solid structure and forms a porous bone graft that has mechanical integrity.

In the following, further features, advantages and embodiments of the synthetic bone substitute according to the first aspect will be explained in detail.

The synthetic bone substitute can be used to fill any kind of cavity, void or recess in a substrate. Due to the bio-compatibility of the materials used for the bone substitute, the bone substitute is specially suited for filling voids, cavities or recesses in living tissue such as bones.

A bio-resorbable polymer may be blended with a bio-ceramic filler and then manufactured to have a porous foam structure. This may be done e.g. during the moulding process. The porous foam structure may have an interconnected pore size that mimics that of human cancellous bone. The porous foam structure may have an interconnected porosity of between 5% and 85%, preferably between 10% and 50%. The pore volume may be between 0,1mm³ and 20mm³, preferably between 0,5mm³ and 5mm³ and more preferably between 1mm³ and 3mm³. In other words, the porous foam structure may be an open-cell foam structure in which neighbouring pores are interconnected. Due to these interconnections, a fluid such as a liquid plasticizer can easily enter the foam structure and wet the entire surface of the foam structure.

The bio-resorbable polymer can be a bio-compatible polymer, i.e. a polymer which is accepted by living tissue such as bones thereby preventing rejection reactions in the body of a patient. Alternatively, the bio-resorbable polymer can be a bio-absorbable polymer, i.e., a polymer which may be absorbed by a human or animals body after a certain period such that at least parts of the foam structure may be replaced by living tissue after this period, thereby providing an increase stability of the connection between an implanted bone substitute and living tissue. Furthermore, rejection reactions can be reduced.

An example of a bio-resorbable material comprises polylactic acid (PLA).

One possible bio-absorbable material comprises a copolymer comprising between 50% and 90% Poly-L-lactide and between 10% and 50% Poly-D, L-lactide. In particular, the bio-absorbable material may be a copolymer comprising 70 weight% Poly-L-lactide and 30 weigh% Poly-D, L-lactide. Preferably, the bio-absorbable material may be formed as an amorphous material.

The above described material may be a suitable material usable for the bone substitute, which material may exhibit a suitable tensile strength of about 60 MPa, and a suitable E-modulus of about 3500 MPa. Furthermore, a bone substitute including the above material, may retain its strength for about a sufficient time when implanted into a human or animals body, Such a time span may be about 16 to 26 weeks. The described copolymer may have a resorption time of about two to three years in a human or animals body. The material may further exhibit an increase of implant volume up to 200% after 24 month from the implantation in the target structure. Such a material may further be easily to be sterilized by γ-radiation. A suitable energy dose may be between 20 kGy and 30 kGy, in particular below 25 kGy.

The bio-ceramic filler material may be used to provide mechanical strength to the porous foam structure. For example, a calcium phosphate from the family of the inorganic calcium phosphate salts may be used for the bio-ceramic filler. For example, tri-calcium-phosphate (TCP, Ca₃(PO₄)₂) may be used. Alternatively hydroxyapatite (Ca₁₀(PO₄)₆(OH)₂), dicalcium phosphate anhydrous CaHPO₄, dicalcium phosphate dihydrate CaHPO₄ * 2H₂O, monocalcium phosphate Ca (H₂PO₄)₂, calcium pyrophosphate Ca₂P₂O₇, octacalcium phosphate Ca₈H₂(PO₄)₆, calcium carbonate CaCO₃, calcium sulphate CaSO₄ or titanium dioxide TiO₂ can be used for the bio-ceramic filler material.

Further fillers or bulking agents can consist of particles containing silver which would have a local anti microbial effect, and/or particles containing strontium ranalate or a bisphosphonate or osteogenic protein either individually or in combinations that result in a net anabolic effect.

The ratio of bio-resorbable polymer mass to bio-ceramic filler mass may range from 70:30 to 97:3

Normally, i.e. without applying any further means, the porous foam structure made from the blend of bio-resorbable polymer and bio-ceramic filler material is a rigid structure which cannot be substantially compressed without irreversible damage to the foam structure. However, the inventor of the present invention has found that the porous foam structure can be treated with a plasticizer thereby softening the porous foam structure. In other words, by applying the plasticizer to the porous foam structure the latter can be softened such that it is compressible to a certain degree. In a softened state, the porous foam structure may have properties of a sponge.

After being softened by the plasticizer the porous foam structure may be compressible from an extended original state with an extended volume to a compressed state with a compressed volume wherein the compressed volume is less than 50 %, preferably less than 30 % and more preferably less than 10 % of the extended volume. Accordingly, the softened foam structure may be compressed to a fraction of its original volume and, in this compressed state, may be introduced in a cavity.

The porous foam structure softened by the plasticizer may have a certain degree of elasticity. In other words, the porous foam structure can be elastically compressed by an external force wherein, due to its elasticity, the foam structure may at least partially restore to its original structure and volume when the external force is released.

The plasticizer may be adapted to dissipate from the porous foam structure within a predetermined period. In other words, the plasticizer may slowly disappear from the foam structure under certain conditions such as when it is subjected to an elevated temperature such as for example the temperature of 37 °C in a human body or when in contact with specific fluids such as for example body fluids like blood or water. When the plasticizer had disappeared from the foam structure the characteristics of the foam structure which are due to the presence of the plasticizer disappear as well. Especially the softening characteristics created by the plasticizer is reduced or disappears and the porous foam structure having substantially no more plasticizer in it becomes rigid such that it can be loaded with external forces without compression of the foam structure. In other words, porous foam structure obtains a higher rigidity after dissipation of the plasticizer.

The plasticizer can be N-Methyl-2-Pyrolidone. Preferably, the plasticizer is absorbed into the porous foam structure and may be homogeneously distributed throughout the synthetic bone substitute rendering the porous foam structure homogeneously compressible.

The porous foam structure has a volume in an expanded state, i.e. a non-compressed state, of between 0.5cm³ and 50cm³, preferably between 1cm³ and 5cm³. Using a bone substitute having such small porous foam structures, a plurality of pieces of bone substitute can be inserted into a cavity and can fill the cavity in an optimum way.

According to a second aspect of the present invention a method for preparing a synthetic bone substitute is described, the method comprising: providing a porous foam structure comprising a bio-resorbable polymer and a bio-ceramic filler material and applying a plasticizer to the porous foam structure for softening the porous foam structure. The application of the plasticizer may e.g. be realised by dipping the porous foam structure into a liquid plasticizer, e.g. for a predetermined period between 2 and 20 minutes, depending upon the volume of the porous foam structure. Alternatively, the porous foam structure may be stored within the liquid plasticizer for a longer period until being actually used in a surgical operation. Therein, the porous foam structure may be soaked with plasticizer.

The method may further comprise the step of compressing the porous foam structure. For this purpose, an external force can be applied to the softened foam structure thereby compressing it using its elasticity.

According to a further aspect of the present invention a method for filling a cavity in substrate is described, the method comprising inserting a synthetic bone substitute according to the above-described first aspect of the invention into the cavity. The method can be used for filling cavities in any kind of substrates. For example, cavities in living tissue such as bones can be filled or cavities in non-living tissue can be filled.

In order to simplify the insertion into the cavity the synthetic bone substitute may be compressed before insertion into the cavities thereby reducing its volume. In this compressed state, the bone substitute can be easily introduced into the cavity. This can be especially advantageous in a case where an opening to the cavity through which the bone substitute is to be inserted has a smaller cross-section than a parallel cross-section within the opening itself. Whereas in former approaches the insertion opening and the cavity itself should have had approximately the same cross-section such that an entire piece of porous foam could have been introduced therein, the compressible bone substitute according to the invention can be introduced via a small opening and then re-expand within the cavity before solidification due to dissipation of the plasticizer.

It has to be noted that embodiments of the invention are described with reference to different subject-matters. In particular, some embodiments are described with reference to apparatus type claims whereas other embodiments are described with reference to method type claims. However, a person skilled in the art will gather from the above and the following description that, unless other notified, in addition to any combination of features belonging to one type of subject-matter also any combination between features relating to the different subject-matters, in particular between features of the apparatus type claims and features of the method type claims, is considered to be disclosed with this application.

The aspects defined above and further aspects, features and advantages of the present invention can be derived from the examples of embodiments described hereinafter.

The invention will be described in more detail hereinafter with reference to examples of embodiments but to which the invention is not limited.

### Brief description of the drawings

Fig. 1 shows an X-ray image illustrating a fracture including a cavity in a human bone.
Fig. 2 schematically shows an arrangement for inserting a synthetic bone substitute into a void in a bone.
Figs. 3a to 3e show different geometries showing how the elements could close pack.

The following examples of experiments made by the inventor show embodiments of methods for preparing the synthetic bone substitute according to the invention.

### Example 1

A cylinder of porous material comprising by volume 95 % PLA and 5 % TCP with an interconnected porosity of 5 - 85 % volume fraction was exposed to N-Methyl-2-Pyrolidone plasticizer for 8 minutes. It was then compressed using hand pressure (less than 15 N) from 13.5 mm to 6.0 mm. It was then exposed under no external pressure to water at 37°C and recovered to 9 mm after 10 minutes. After one hour it had recovered its original mechanical properties.

### Example 2

A block of porous material comprising by volume 50 % PLA and 50 % CaP with intercon-nected porosity of 5 - 85 % was provided. This material produced using 3-D printing of alternating layers of a CaP and a bio-degradable polymer. It was then compressed using hand pressure (less than 150 N). The external applied force was then removed and the material was allowed to recover to its original dimensions properties.

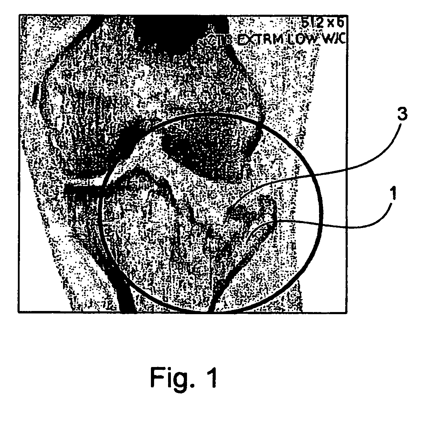

Fig. 1 shows an X-ray image of a bone 1 with a cavity 3 due to a fracture.

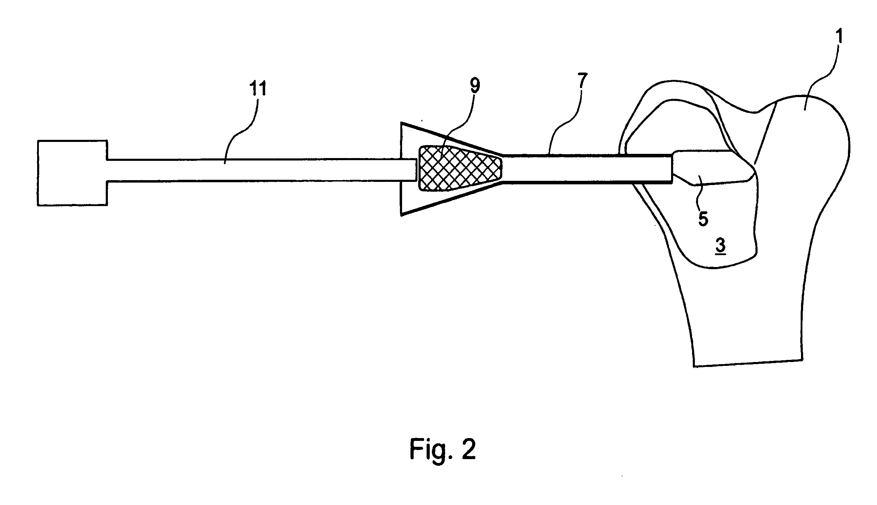

In Fig. 2, the bone 1 is represented schematically with its cavity 3. The cavity 3 has an opening hole 5 the cross-section of which is substantially smaller than the cross-section of the cavity 3. A funnel 7 can be introduced into the cavity 3 via the opening 5. A porous synthetic bone substitute substrate 9 that has been previously softened by dipping it into a liquid plasticizer such that the plasticizer is partly absorbed in the porous foam structure of the bone substitute is introduced into the funnel 7. Then, the bone substitute substrate 9 is pushed through the funnel with a pusher tool 11. As the bone substitute substrate 9 originally has a larger cross-section than at the narrowest portion of the funnel the bone substitute substrate 9 is compressed while being pushed through the funnel 9. Finally, the bone substitute substrate 9 reaches the opening 5 and is inserted into the cavity 3. It falls into the cavity 3 and accordingly the compression force exerted by the funnel 7 is released. Therefore, the elastic bone substitute substrate 9 can restore its original geometry after a while. Preferably during this while further bone substitute substrates were inserted into the bone cavity 3 until it is completely filled. While trying to restore their original geometry the plurality of bone substitute substrates 9 will try to expand and will therefore fill remaining gaps between the bone substitute substrates thereby completely filling the bone cavity 3. After a further while the plasticizer will have disappeared from the bone substitute substrates 9 as it is absorbed by surrounding liquids such as blood or water. Therefore, the synthetic bone substitute substrates will re-solidify and form a loadable filling for the cavity 3 which due to its porosity is lightweight and due to the rigidity of the blend of bio-ceramic filler and bio-resorbable polymer can support heavy loads.

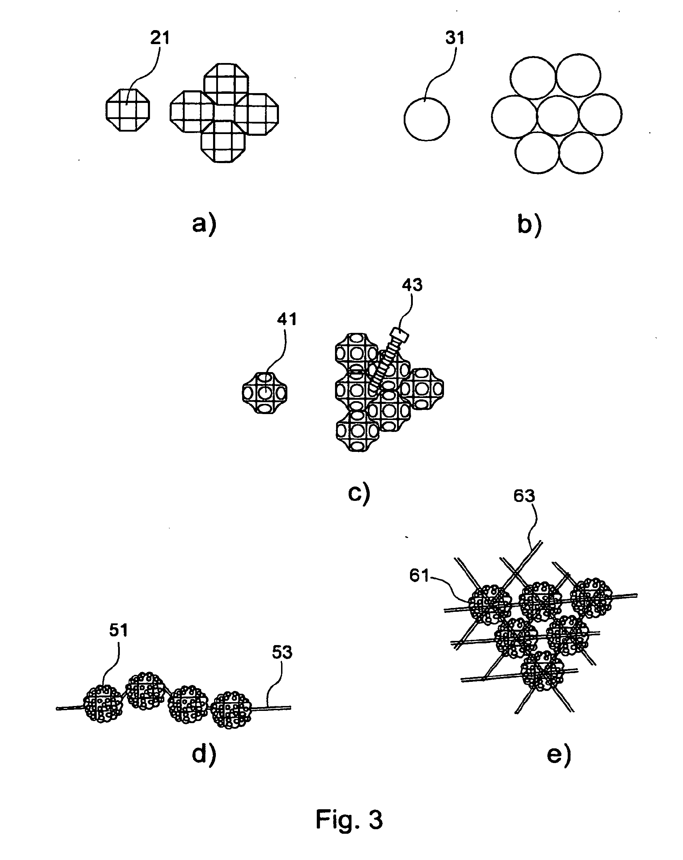

Figs. 3a to 3e show different geometries of synthetic bone substitute substrates and their possible arrangements.

Fig. 3a shows synthetic bone substitute substrates 21 having an octagonal cross-section. These substrates 21 can be arranged to form a compact packing.

Fig. 3b shows approximately spherical bone substitute substrates 31 which can be packed in a sphere packing.

Fig. 3c shows a specially structured synthetic bone substitute substrate 41 into which for example a screw 43 can be screwed in. The specially structured synthetic bone substitute substrate in this case has been formed into elements whose external geometry favours interlocking with neighbouring elements and whose internal geometry has been formed with perforating holes that facilitate the insertion of screws. The addition of the screws is intended either to add additional stability to the combined elements or to secure them to adjacent bone.

Fig. 3d shows bone substitute substrates 51 arranged along a filament 53. In Fig. 3e, a plurality of bone substitute substrates 61 are arranged coupled by a mesh of filaments 63. In these embodiments the substrate elements have been connected using threads or cables that act as a means of connecting the elements. This has the advantage that a number of smaller substrate elements may be combined into a larger construct to avoid that the individual elements becoming detached or migrating within the void to be filled.

The invention may be summarized as follows: A synthetic bone substitute (9) is disclosed comprising a porous foam structure comprising a bio-resorbable polymer and a bio-ceramic filler material wherein a plasticizer is used for softening the porous foam structure such that the synthetic bone substitute may be compressed by an external force. Due to its elasticity, the synthetic bone substitute may restore its original form after releasing the external force. Furthermore, due to dissipation of the plasticizer, the porous foam structure can attain substantial rigidity thereby functioning as lightweight, stable bone substitute. Such compressible bone substitute can be compressed, then inserted through a small opening hole to a cavity and once in a cavity restore its original volume and rigidity.

It should be noted that the term "comprising" does not exclude other elements or steps and the "a" or "one" does not exclude a plurality. Also elements described in association with different embodiments and aspects may be combined. It should also be noted that reference signs in the claims shall not be construed as limiting the scope of the claims.

### List of reference numerals:

- 1: bone
- 3: cavity
- 5: opening hole
- 7: funnel
- 9: bone substitute substrate
- 11: pushing tool
- 21, 31, 41, 51, 61: bone substitute substrates
- 43: screw
- 53, 63: filament

## Claims

1. A synthetic bone substitute (9) comprising:
a porous foam structure comprising a bioresorbable polymer and a bioceramic filler material; and
a plasticiser for softening the porous foam structure,
wherein the plasticiser is adapted for softening the porous foam structure such that it is compressible ,
wherein the porous foam structure has a volume in an expanded state of between 0.5cm³ and 50cm³,
wherein the porous foam structure softened by the plasticiser is compressible from an extended state with an extended volume to a compressed state with a compressed volume, the compressed volume being less than 50% of the extended volume.

2. The synthetic bone substitute according claim 1,
wherein the porous foam structure softened by the plasticiser has a degree of elasticity.

3. The synthetic bone substitute according to one of the preceding claims,
wherein the plasticiser is adapted to dissipate from the porous foam structure within a predetermined period.

4. The synthetic bone substitute according to claim 3,
wherein the porous foam structure is adapted to obtain a higher rigidity after dissipation of the plasticiser.

5. The synthetic bone substitute according to one of the preceding claims,
wherein the plasticiser is absorbed into the porous foam structure.

6. The synthetic bone substitute according to one of the preceding claims,
wherein the plasticiser is N-Methyl-2-Pyrolidone.

7. The synthetic bone substitute according to one of the preceding claims,
wherein the bioresorbable material comprises Poly Lactic Acid.

8. The synthetic bone substitute according to one of the preceding claims,
wherein the bioceramic filler comprises a calcium phosphate from the family of the inorganic calcium phosphate salts.

9. A method for preparing a synthetic bone substitute, the method comprising:
providing a porous foam structure comprising a bioresorbable polymer and a bioceramic filler material; and
applying a plasticiser to the porous foam structure for softening the porous foam structure,
wherein the plasticiser is adapted for softening the porous foam structure such that it is compressible,
wherein the porous foam structure softened by the plasticiser is compressible from an extended state with an extended volume to a compressed state with a compressed volume, the compressed volume being less than 50% of the extended volume, and
wherein the porous foam structure has a volume in an expanded state of between 0.5cm³ and 50cm³.

10. The method for preparing a synthetic bone substitute according to claim 9, further comprising compressing the porous foam structure.

11. A method for filling a cavity in a substrate not being part of a living human or animal body, the method comprising:
inserting a synthetic bone substitute according to any of claims 1 to 8 into the cavity.

12. The method according to claim 11,
wherein the synthetic bone substitute is compressed before insertion into the cavity.

13. The method according to claim 11 or 12,
wherein the synthetic bone substitute is inserted to the cavity via an opening the opening having a cross section being smaller than a parallel cross section of the cavity.

## Patentansprüche

1. Synthetisches Knochenersatzmaterial (9), das Folgendes aufweist:
eine poröse Schaumstruktur, die ein bioresorbierbares Polymer und ein biokeramisches Füllmaterial enthält; und
ein Plastifizierungsmittel zum Weichmachen der porösen Schaumstruktur,
wobei das Plastifizierungsmittel zum Weichmachen der porösen Schaumstruktur derart angepasst ist, das diese komprimierbar ist,
wobei die poröse Schaumstruktur in einem ausgedehnten Zustand ein Volumen von zwischen 0,5 cm³ und 50 cm³ aufweist,
wobei die poröse Schaumstruktur, die durch das Plastifizierungsmittel weich gemacht ist, von einem ausgedehnten Zustand mit einem ausgedehnten Volumen in einen komprimierten Zustand mit einem komprimierten Volumen komprimierbar ist, wobei das komprimierte Volumen weniger als 50 % des ausgedehnten Volumens beträgt.

2. Synthetisches Knochenersatzmaterial nach Anspruch 1,
wobei die poröse Schaumstruktur, die durch das Plastifizierungsmittel weich gemacht ist, einen Plastizitätsgrad aufweist.

3. Synthetisches Knochenersatzmaterial nach einem der vorangehenden Ansprüche,
wobei das Plastifizierungsmittel angepasst ist, sich innerhalb einer vorgegebenen Zeitspanne aus der porösen Schaumstruktur zu verflüchtigen.

4. Synthetisches Knochenersatzmaterial nach Anspruch 3,
wobei die poröse Schaumstruktur angepasst ist, nach dem Verflüchtigen des Plastifizierungsmittels eine höhere Festigkeit zu erlangen.

5. Synthetisches Knochenersatzmaterial nach einem der vorangehenden Ansprüche,
wobei das Plastifizierungsmittel in der porösen Schaumstruktur absorbiert ist.

6. Synthetisches Knochenersatzmaterial nach einem der vorangehenden Ansprüche,
wobei das Plastifizierungsmittel N-Methyl-2-Pyrolidon ist.

7. Synthetisches Knochenersatzmaterial nach einem der vorangehenden Ansprüche,
wobei das bioresorbierbare Material Polymilchsäure umfasst.

8. Synthetisches Knochenersatzmaterial nach einem der vorangehenden Ansprüche,
wobei das biokeramische Füllmaterial ein Calciumphosphat aus der Familie der anorganischen Calciumphosphatsalze enthält.

9. Verfahren zur Herstellung eines synthetischen Knochenersatzmaterials, wobei das Verfahren Folgendes umfasst:
Bereitstellen einer porösen Schaumstruktur, die ein bioresorbierbares Polymer und ein biokeramisches Füllmaterial enthält; und
Anwenden eines Plastifizierungsmittels auf die poröse Schaumstruktur zum Weichmachen der porösen Schaumstruktur,
wobei das Plastifizierungsmittel zum Weichmachen der porösen Schaumstruktur derart angepasst ist, das diese komprimierbar ist,
wobei die poröse Schaumstruktur, die durch das Plastifizierungsmittel weich gemacht ist, von einem ausgedehnten Zustand mit einem ausgedehnten Volumen in einen komprimierten Zustand mit einem komprimierten Volumen komprimierbar ist, wobei das komprimierte Volumen weniger als 50 % des ausgedehnten Volumens beträgt, und
wobei die poröse Schaumstruktur in einem ausgedehnten Zustand ein Volumen von zwischen 0,5 cm³ und 50 cm³ aufweist.

10. Verfahren zur Herstellung eines synthetischen Knochenersatzmaterials nach Anspruch 9,
das ferner das Komprimieren der porösen Schaumstruktur umfasst.

11. Verfahren zum Füllen eines Hohlraumes in einem Substrat, das nicht Bestandteil eines lebenden menschlichen oder tierischen Körpers ist, wobei das Verfahren Folgendes umfasst:
Einsetzen eines synthetischen Knochenersatzmaterials nach einem der Ansprüche 1 bis 8 in den Hohlraum.

12. Verfahren nach Anspruch 11,
wobei das synthetische Knochenersatzmaterial vor dem Einsetzen in den Hohlraum komprimiert wird.

13. Verfahren nach Anspruch 11 oder 12,
wobei das synthetische Knochenersatzmaterial über eine Öffnung in den Hohlraum eingesetzt wird, wobei die Öffnung einen Querschnitt aufweist, der kleiner als ein paralleler Querschnitt des Hohlraums ist.

## Revendications

1. Substitut osseux synthétique (9) comprenant :
une structure en mousse poreuse comprenant un polymère biorésorbable et un matériau de remplissage biocéramique ; et
un plastifiant pour ramollir la structure en mousse poreuse,
dans lequel le plastifiant étant adapté pour ramollir la structure en mousse poreuse de manière à ce qu'elle soit compressible,
dans lequel la structure en mousse poreuse ayant un volume dans un état expansé entre 0,5 cm³ et 50 cm³,
dans lequel la structure en mousse poreuse ramollie par le plastifiant étant compressible d'un état expansé avec un volume expansé à un état comprimé avec un volume comprimé, le volume comprimé étant inférieur à 50 % du volume expansé.

2. Substitut osseux synthétique selon la revendication 1,
dans lequel la structure en mousse poreuse ramollie par le plastifiant ayant un degré d'élasticité.

3. Substitut osseux synthétique selon l'une quelconque des revendications précédentes,
dans lequel le plastifiant étant adapté pour se dissiper de la structure en mousse poreuse au cours d'une période prédéterminée.

4. Substitut osseux synthétique selon la revendication 3,
dans lequel la structure en mousse poreuse est adaptée pour obtenir une rigidité supérieure après la dissipation du plastifiant.

5. Substitut osseux synthétique selon l'une quelconque des revendications précédentes,
dans lequel le plastifiant étant absorbé dans la structure en mousse poreuse.

6. Substitut osseux synthétique selon l'une quelconque des revendications précédentes,
dans lequel le plastifiant étant la N-méthyl-2-pyrrolidone.

7. Substitut osseux synthétique selon l'une quelconque des revendications précédentes,
dans lequel le matériau biorésorbable comprenant de l'acide polylactique.

8. Substitut osseux synthétique selon l'une quelconque des revendications précédentes,
dans lequel le matériau de remplissage biocéramique comprenant du phosphate de calcium de la famille des sels de phosphate de calcium inorganiques.

9. Procédé de préparation d'un substitut osseux synthétique, le procédé comprenant :
la fourniture d'une structure en mousse poreuse comprenant un polymère biorésorbable et un matériau de remplissage biocéramique ; et
l'application d'un plastifiant sur la structure en mousse poreuse pour ramollir la structure en mousse poreuse,
dans lequel le plastifiant étant adapté pour ramollir la structure en mousse poreuse de manière à ce qu'elle soit compressible,
la structure en mousse poreuse ramollie par le plastifiant étant compressible d'un état expansé avec un volume expansé à un état comprimé avec un volume comprimé, le volume comprimé étant inférieur à 50 % du volume expansé, et
dans lequel la structure en mousse poreuse ayant un volume dans un état expansé entre 0,5 cm³ et 50 cm³.

10. Procédé de préparation d'un substitut osseux synthétique selon la revendication 9, comprenant en outre la compression de la structure en mousse poreuse.

11. Procédé de remplissage d'une cavité dans un substrat qui ne fait pas partie d'un corps humain ou animal vivant, le procédé comprenant :
l'insertion d'un substitut osseux synthétique selon l'une quelconque des revendications 1 à 8 dans la cavité.

12. Procédé selon la revendication 11,
dans lequel le substitut osseux synthétique est comprimé avant l'insertion dans la cavité.

13. Procédé selon la revendication 11 ou 12,
dans lequel le substitut osseux synthétique est inséré dans la cavité via une ouverture, l'ouverture ayant une section transversale qui est plus petite qu'une section transversale parallèle de la cavité.
